# EUROPÄISCHE PATENTSCHRIFT

(11) **EP 0 948 957 B1**
(45) Veröffentlichungstag und Bekanntmachung des Hinweises auf die Patenterteilung: **15.03.2006**
(21) Anmeldenummer: 99104860.4
(22) Anmeldetag: 11.03.1999
(51) Int. Cl.: A61K 8/81, C08F 222/04

(54) **Verwendung von Copolymerisaten monoethylenisch ungesättigter Carbonsäuren als Solubilisatoren**
Use of copolymers of monoethylenically unsaturated carboxylic acids as solubiliser
Usage de copolymères d'acides carboxyliques insaturés monoéthyléniquement comme solubilisants

(30) Priorität: 18.03.1998 DE 19811919
(43) Veröffentlichungstag der Anmeldung: 13.10.1999
(73) Patentinhaber: BASF Aktiengesellschaft, 67056 Ludwigshafen (DE)
(72) Erfinder: Meffert, Helmut, Dr., 68161 Mannheim (DE); Sanner, Axel, Dr., 67227 Frankenthal (DE); Berndl, Gunther, Dr., 67273 Herxheim (DE); Ruchatz, Folker, Dr., 67433 Neustadt (DE); Kolter, Karl, Dr., 67117 Limburgerhof (DE); Bauer, Kurt, Heinz, Prof., Dr., 79112 Freiburg (DE)

(56) Entgegenhaltungen:
- EP-A- 0 268 164
- EP-A- 0 494 022
- WO-A-93/22357
- US-A- 3 915 921
- US-A- 4 911 736

## Beschreibung

Die Erfindung betrifft die Verwendung von Copolymerisaten monoethylenisch ungesättigter Carbonsäuren als Solubilisatoren

Bei der Herstellung homogener pharmazeutischer oder kosmetischer Zubereitungen hat die Solubilisierung von hydrophoben Stoffen eine sehr große praktische Bedeutung erlangt.

Unter Solubilisierung ist eine Löslichkeitsverbesserung durch oberflächenaktive Verbindungen zu verstehen, die in der Lage sind, schlecht wasserlösliche oder wasserunlösliche Stoffe in klare, höchstens opaleszierende wäßrige Lösungen zu überführen, ohne daß hierbei die chemische Struktur dieser Stoffe eine Veränderung erfährt.

Die hergestellten Solubilisate sind dadurch gekennzeichnet, daß der schlecht wasserlösliche oder wasserunlösliche Stoff in den Molekülassoziaten der oberflächenaktiven Verbindungen, die sich in wäßriger Lösung bilden - den sogenannten Mizellen - gelöst vorliegt. Die resultierenden Lösungen sind stabile einphasige Systeme, die optisch klar bis opaleszent erscheinen und ohne Energieeintrag hergestellt werden können.

Solubilisatoren können beispielsweise das Aussehen von kosmetischen Formulierungen sowie von Lebensmittelzubereitungen verbessern, indem sie die Formulierungen transparent machen. Außerdem kann im Falle von pharmazeutischen Zubereitungen auch die Bioverfügbarkeit und damit die Wirkung von Arzneistoffen durch die Verwendung von Solubilisatoren gesteigert werden.

Als Solubilisatoren für pharmazeutische Arzneistoffe und kosmetische Wirkstoffe werden hauptsächlich folgende Produkte eingesetzt:
- ethoxiliertes (hydriertes) Ricinusöl, (z.B. Cremophor® Marken, Fa. BASF);
- ethoxilierte Sorbitanfettsäureester, (z.B. Tween® Marken, Fa. ICI);
- ethoxilierte Hydroxystearinsäure, (z.B. Solutol® Marken, Fa. BASF).

Die oben beschriebenen, bisher eingesetzten Solubilisatoren zeigen jedoch eine Reihe anwendungstechnischer Nachteile.

So ist z. B. deren parenterale Applikation mit einer Freisetzung von Histamin und einem daraus resultierenden Blutdruckabfall verbunden (Lorenz et al., Agents and Actions, Vol. 12, 1/2, 1982).

Die bekannten Solubilisatoren besitzen für einige schwerlösliche Arzneistoffe wie z.B. Clotrimazol nur eine geringe lösungsvermittelnde Wirkung.

Oberflächenaktive Verbindungen besitzen häufig eine hohe hämolytische Aktivität, die einer Anwendung auf dem Gebiet der Pharmazie, insbesondere in Parenteralia entgegensteht.

In der Dissertation von G. Eisenmann (Prof. Bauer, Universität Freiburg, 1994, Seite 44/45, 93 bis 95) wird die polymeranaloge Umsetzung von Polyacrylsäure mit einem M_{W} ≈ 2000 mit linearen Alkylaminen der Kettenlänge C₆ bis C₁₈ unter Aktivierung der Carboxylgruppen mit Dicyclohexylcarbodiimid zu den entsprechenden Amiden beschrieben. Der Anteil an amidierten Carboxylgruppen beträgt zwischen 20% und 33%. Die hier als Solubilisatoren beschriebenen Copolymere haben jedoch den Nachteil, daß sie bei Konzentrationen > 5 Gew.-% nicht mehr ausreichend wasserlöslich sind.

C. Tribet et al. (Proc. Natl. Acad. Sci. 1996, 93, 15047-15050) beschreiben mit Octylamin und Isopropylamin polymeranalog umgesetzte Polyacrylsäure mit einem Molekulargewicht von M_{W} ≈ 8000 bis 34000. Aufgrund ihres Eigenschaftsprofils (ungenügende Wasserlöslichkeit, zu geringe lösungsvermittelnde Wirkung) sind diese Polymere für die Verwendung als Solubilisatoren in pharmazeutischen und kosmetischen Zubereitungen nicht zufriedenstellend.

US 4,432,881 beschreibt hydrophob modifizierte Polyacrylsäure mit einem Molekulargewicht zwischen 200000 und 5000000, die durch Copolymerisation von Acrylsäure mit den entsprechenden N-Alkylacrylamiden oder Acrylaten erhalten werden. Verwendet werden die erhaltenen Polymere als dispergierbare hydrophobe Verdicker.

In US 4,395,524 wird die Copolymerisation von hydrophilen Komponenten (z.B. Acrylamid, Acrylsäure, N-Vinylpyrrolidon u.a.) mit N-Alkylacrylamiden beschrieben. Die so erhaltenen Polymere mit einem Molekulargewicht von 30000 bis 2000000 werden als Verdicker, Sedimentationsstabilisatoren oder Dispergiermittel verwendet.

EP-A-0 268 164 beschreibt die Verwendung von Copolymerisaten von monoolefinisch ungesättigten Säuren und Alkylester monoolefinisch ungesättigter Säuren zur Stabilisierung von O/W-Emulsionen.

Es bestand nun die Aufgabe, neue Solubilisatoren für pharmazeutische, kosmetische sowie lebensmitteltechnische Anwendungen bereitzustellen, die die oben genannten Nachteile nicht aufweisen.

Diese Aufgabe wurde gelöst durch die Verwendung von Copolymeren, enthaltend
a) 85 bis 95 Mol-% mindestens einer monoethylenisch ungesättigten C₃-C₈-Carbonsäure;
b) 0,1 bis 15 Mol-% mindestens eines Monomeren ausgewählt aus der Gruppe
   b₁) N-C₁₂-C₂₂-Alkyl-substituierter Amide von monoethylenisch ungesättigten C₃-C₈-Carbonsäuren,
   b₂) N,N-C₁₂-C₂₂-Dialkyl-substituierter Amide von monoethylenisch ungesättigten C₃-C₈-Carbonsäuren,
c) 0 bis 14,9 Mol-% mindestens eines Monomeren ausgewählt aus der Gruppe der
   c₁) Vinylester von aliphatischen C₈-C₁₈-Carbonsäuren,
   c₂) C₈-C₁₈-Alkyl-Vinylether,
wobei sich die Mol-% Angaben der Einzelkomponenten zu 100% addieren, als Solubilisator.

Als hydrophile Komponente a) seien folgende copolymerisierbare Monomere genannt:

Monoethylenisch ungesättigte Carbonsäuren mit 3 bis 8 C-Atomen, wie z.B. Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure.

Aus dieser Gruppe von Monomeren werden bevorzugt Acrylsäure, Methacrylsäure, Maleinsäure oder Mischungen der genannten Carbonsäuren verwendet.

Die monoethylenisch ungesättigten Carbonsäuren können als freie Säure, als Anhydride sowie in partiell oder vollständig neutralisierter Form bei der Copolymerisation eingesetzt werden.

Für die Neutralisation der oben genannten Carbonsäuren verwendet man vorzugsweise Alkalimetall- oder Erdalkalimetallbasen, Ammoniak oder Amine, bevorzugt Natronlauge, Kalilauge, Soda, Pottasche, Natriumhydrogencarbonat, Magnesiumoxid, Calciumhydroxid, Calciumoxid, gasförmiges oder wäßriges Ammoniak, Triethylamin, Ethanolamin, Diethanolamin, Triethanolamin, Morpholin, Diethylentriamin oder Tetra-ethylenpentamin.

Es ist selbstverständlich auch möglich, Mischungen der genannten Monomeren einzusetzen.

Der Anteil der hydrophilen Monomerbausteine a) im Copolymerisat liegt im Bereich von 85 bis 95 Mol-%, besonders bevorzugt im Bereich von 87 bis 93 Mol-%.

Als hydrophobe Komponenten b) seien folgende polymerisierbare Comonomere genannt:

N-C₁₂-C₂₂-Alkyl- oder N,N-C₁₂-C₂₂-Dialkyl-substituierte Amide von monoethylenisch ungesättigten C₃-C₈-Carbonsäuren, wobei es sich bei den Alkylresten um aliphatische oder cycloaliphatische Alkylreste mit 12 bis 22 Kohlenstoffatomen handelt.

Unter den monoethylenisch ungesättigten Carbonsäuren mit 3 bis 8 C-Atomen sind die bereits oben genannten Säuren, bevorzugt Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure zu verstehen.

Aus dieser Gruppe von Monomeren werden ebenfalls besonders bevorzugt Acrylsäure, Methacrylsäure, Maleinsäure oder Mischungen der genannten Carbonsäuren verwendet.

Bevorzugte amidierte Comonomere sind beispielsweise N-Stearylacrylamid, N-Stearylmethacrylamid, N-(1-Methyl)undecylacrylamid, N-(1-Methyl)undecylmethacrylamid, N-Dodecylacrylamid, N-Dodecylmethacrylamid, N-Cetylacrylamid, N-Cetylmethacrylamid, N-Dodecylacrylamid, N-Dodecylmethacrylamid, N-Myristylacrylamid, N-Myristylmethacrylamid.

Im Falle von Maleinsäureanhydrid als Comonomer kann diese polymeranalog mit N-Alkylaminen durch Ringöffnung zu den entsprechenden Amiden umgesetzt werden.

Der Anteil der hydrophoben Monomerbausteine b) im Copolymerisat liegt im Bereich von 0,1 bis 15 Mol-%, besonders bevorzugt im Bereich von 0,1 bis 13 Mol-%.

Als weitere zusätzliche Komponente c) können gegebenenfalls Vinylester langkettiger aliphatischer, gesättigter oder ungesättigter C₈-C₁₈-Carbonsäuren, wie z.B. Caprylsäure, Caprinsäure, Laurinsäure, Myristinsäure, Palmitinsäure, Stearinsäure, Arachinsäure, eingesetzt werden.

Ferner können C₈-C₁₈-Alkyl-Vinylether einpolymerisiert werden.

Als bevorzugte Alkylreste der Vinylether seien verzweigte oder unverzweigte C₈-C₁₈-Alkylketten wie z.B. n-Octyl, 2-Ethylhexyl, n-Nonyl, n-Decyl, n-Undecyl, n-Dodecyl, n-Tridecyl, n-Tetradecyl, n-Pentadecyl, n-Hexadecyl, n-Heptadecyl sowie n-Octadecyl genannt.

Der Anteil der hydrophoben Monomerbausteine c) im Copolymerisat liegt im Bereich von 0 bis 14,9 Mol-%, besonders bevorzugt im Bereich von 0 bis 12,9 Mol-%.

Selbstverständlich können auch Mischungen der jeweiligen Monomeren aus den Gruppen a) bis c) polymerisiert werden.

Unter Umständen kann es sinnvoll sein, neben den bereits genannten Monomerbausteinen a) bis c) weitere geeignete Comonomere, beispielsweise monoethylenisch ungesättigte C₃-C₈-Carbonsäureester von kurzkettigen C₁-C₄-Alkoholen, Amide und Nitrile in Anteilen von 0 bis 5 Mol-% für die Polymerisation zu verwenden.

Beispielhaft seien genannt: Acrylsäuremethylester, Acrylsäureethylester, Methacrylsäuremethylester, Methacrylsäureethylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Acrylamid, Methacrylamid, N-Dimethylacrylamid, N-tert.-butylacrylamid, Acrylnitril, Methacrylnitril, Dimethylaminoethylacrylat, Diethyl-aminoethylacrylat, Diethylaminoethylmethacrylat sowie die Salze der zuletzt genannten Monomeren mit Carbonsäuren oder Mineralsäuren sowie die quaternierten Produkte.

Die erfindungsgemäßen Copolymere haben ein Molekulargewicht von 1000 bis 30000 g/mol, bevorzugt 2000 bis 20000 g/mol, besonders bevorzugt 3000 bis 8000 g/mol.

### Anwendungen:

Durch die vorliegende Erfindung werden amphiphile Verbindungen für die Anwendung als Lösungsvermittler für pharmazeutische und kosmetische Zubereitungen sowie für Lebensmittelzubereitungen zur Verfügung gestellt. Sie besitzen die Eigenschaft, schwer lösliche Wirkstoffe auf dem Gebiet der Pharmazie und Kosmetik, schwerlösliche Nahrungsergänzungsmittel, beispielsweise Vitamine und Carotinoide aber auch schwerlösliche Wirkstoffe für den Einsatz in Pflanzenschutzmitteln sowie veterinärmedizinische Wirkstoffe zu solubilisieren.

Überraschend wurde bei den beanspruchten Verbindungen ein gutes Solubilisationsvermögen für pharmazeutische und kosmetische Wirkstoffe gefunden. Ferner werden mit den beanspruchten Verbindungen Anwendungen erhalten, die sich durch eine sehr geringe Hämolyserate, einer nebenwirkungsfreien Verträglichkeit nach parenteraler, oraler und topischer Applikation auf Haut- und Schleimhaut auszeichnen. Die Verbindungen besitzen insbesondere keine Nebenwirkungen durch Wechselwirkungen mit Blutkörperchenmembranen. Nach parenteraler Applikation findet keine bzw. nur eine geringe Histaminfreisetzung statt. Die Solubilisatoren sind aufgrund ihres geringen Molekulargewichts nierengängig.

### Solubilisatoren für Kosmetik:

Die erfindungsgemäßen Copolymere können als Solubilisatoren in kosmetischen Formulierungen eingesetzt werden. Beispielsweise eignen sie sich als Solubilisatoren für kosmetische Öle. Sie besitzen ein gutes Solubilisiervermögen für Fette und Öle, wie Erdnußöl, Jojobaöl, Kokosnußöl, Mandelöl, Olivenöl, Palmöl, Ricinusöl, Sojaöl oder Weizenkeimöl oder für etherische Öle wie Latschenkiefernöl, Lavendelöl, Rosmarinöl, Fichtennadelöl, Kiefernnadelöl, Eukalyptusöl, Pfefferminzöl, Salbeiöl, Bergamottöl, Terpentinöl, Melissenöl, Salbeiöl, Wacholderöl, Zitronenöl, Anisöl, Kardamonöl; Pfeffernninzöl, Campheröl etc. oder für Mischungen aus diesen Ölen.

Weiterhin können die erfindungsgemäßen Polymere als Solubilisatoren für in Wasser schwerlösliche oder unlösliche UV-Absorber wie beispielsweise 2-Hydroxy-4-methoxybenzophenon (Uvinul® M 40, Fa. BASF), 2,2`,4,4`-Tetrahydroxybenzophenon (Uvinul® D 50), 2,2`-Dihydroxy-4,4`-dimethoxybenzophenon (Uvinul® D49), 2,4-Dihydroxybenzophenon (Uvinul® 400), 2-Cyano-3,3-diphenylacrylsäure-2'-ethylhexylester (Uvinul® N 539), 2,4,6-Trianilino-p-(carbo-2'-ethylhexyl-1'-oxi)-1,3,5-triazin (Uvinul® T 150), 3-(4-Methoxybenzyliden)-campher (Eusolex® 6300, Fa. Merck), N,N-Dimethyl-4-aminobenzoesäure-2-ethylhexylester (Eusolex® 6007), Salicylsäure-3,3,5-trimethylcyclohexylester, 4-Isopropyldibenzoylmethan (Eusolex® 8020), p-Methoxyzimtsäure-2-ethylhexylester und p-Methoxyzimtsäure-2-isoamylester sowie Mischungen davon verwendet werden.

Gegenstand der vorliegenden Erfindung sind daher auch kosmetische Zubereitungen, die mindestens einen der erfindungsgemäßen Copolymere der eingangs genannten Zusammensetzung als Solubilisatoren enthalten. Bevorzugt sind solche Zubereitungen, die neben dem Solubilisator einen oder mehrere schwerlösliche kosmetische Wirkstoffe, beispielsweise die oben genannten Öle oder UV-Absorber enthalten.

Bei diesen Formulierungen handelt es sich um Solubilisate auf Wasser oder Wasser/Alkohol Basis. Die erfindungsgemäßen Solubilisatoren werden im Verhältnis von 0,2:1 bis 20:1, bevorzugt 1:1 bis 15:1, besonders bevorzugt 2:1 bis 12:1 zum schwerlöslichen kosmetischen Wirkstoff eingesetzt.

Der Gehalt an erfindungsgemäßem Solubilisator in der kosmetischen Zubereitung liegt, abhängig vom Wirkstoff, im Bereich von 1 bis 50 Gew.-%, bevorzugt 3 bis 40 Gew.-%, besonders bevorzugt 5 bis 30 Gew.-%.

Zusätzlich können dieser Formulierung weitere Hilfsstoffe zugesetzt werden, beispielsweise nichtionische, kationische oder anionische Tenside wie Alkylpolyglycoside, Fettalkoholsulfate, Fettalkoholethersulfate, Alkansulfonate, Fettalkoholethoxilate, Fettalkoholphosphate, Alkylbetaine, Sorbitanester, POE-Sorbitanester, Zuckerfettsäureester, Fettsäurepolyglycerinester, Fettsäurepartialglyceride, Fettsäurecarboxylate, Fettalkoholsulfosuccinate, Fettsäuresarcosinate, Fettsäureisethionate, Fettsäuretaurinate, Zitronensäureester, Silikon-Copolymere, Fettsäurepolyglykolester, Fettsäureamide, Fettsäurealkanolamide, quartäre Ammoniumverbindungen, Alkylphenoloxethylate, Fettaminoxethylate, Cosolventien wie Ethylenglykol, Propylenglykol, Glycerin u.a..

Als weitere Bestandteile können natürliche oder synthetische Verbindungen, z.B. Lanolinderivate, Cholesterinderivate, Isopropylmyristat, Isopropylpalmitat, Elektrolyte, Farbstoffe, Konservierungsmittel, Säuren (z.B. Milchsäure, Zitronensäure) zugesetzt werden.

Diese Formulierungen finden beispielsweise in Badezusatzpräparaten wie Badeölen, Rasierwässern, Gesichtswässern, Mundwässern, Haarwässern, Eau de Cologne, Eau de Toilette sowie in Sonnenschutzmitteln Verwendung.

### Beschreibung der Solubilisierungsmethode:

Bei der Herstellung der Solubilisate für kosmetische Formulierugen können die erfindungsgemäßen Copolymere als 100%ige Substanz oder bevorzugt als wäßrige Lösung eingesetzt werden.

Üblicherweise wird der Solubilisator in Wasser gelöst und mit dem jeweils zu verwendenden schwerlöslichen kosmetischen Wirkstoff intensiv vermischt.

Es kann aber auch der Solubilisator mit dem jeweils zu verwendenden schwerlöslichen kosmetischen Wirkstoff intensiv vermischt werden und anschließend unter ständigem Rühren mit demineralisiertem Wasser versetzt werden.

### Solubilisatoren für pharmazeutische Anwendungen:

Die beanspruchten Copolymerisate eignen sich ebenso für die Verwendung als Solubilisator in pharmazeutischen Zubereitungen jeder Art, die dadurch gekennzeichnet sind, daß sie einen oder mehrere in Wasser schwer lösliche oder wasserunlösliche Arzneistoffe sowie Vitamine und/oder Carotinoide enthalten können. Insbesondere handelt es sich dabei um wäßrige Lösungen bzw. Solubilisate zur oralen oder besonders bevorzugt zur parenteralen Applikation, wie z.B. Injektionslösungen zur intravenösen, intramuskulären oder subkutaner oder intraperitonealer Applikation.

Des weiteren eignen sich die beanspruchten Copolymere zum Einsatz in oralen Darreichungsformen wie Tabletten, Kapseln, Pulvern, Lösungen. Hier können Sie den schwerlöslichen Arzneistoff mit einer erhöhten Bioverfügbarkeit zur Verfügung stellen.

Bei der parenteralen Applikation können neben Solubilisaten auch Emulsionen, beispielsweise Fettemulsionen eingesetzt werden. Auch für diesen Zweck eignen sich die beanspruchten Copolymere um einen schwerlöslichen Arzneistoff zu verarbeiten.

Pharmazeutische Formulierungen der oben genannten Art können durch Verarbeiten der beanspruchten Copolymere mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolventien, Stabilisatoren, Konservierungsmittel besonders aufgeführt.

Die verwendeten pharmazeutischen Wirkstoffe sind in Wasser unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Als Beispiele seien hier Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Eine mögliche Herstellvariante ist das Auflösen des Solubilisators in der wäßrigen Phase, gegebenenfalls unter leichtem Erwärmen und das anschließende Lösen des Wirkstoffs in der wäßrigen Solubilisatorlösung. Das gleichzeitige Auflösen von Solubilisator und Wirkstoff in der wäßrigen Phase ist ebenfalls möglich.

Die Verwendung der erfindungsgemäßen Copolymere als Lösungsvermittler kann beispielsweise auch in der Weise erfolgen, daß der wirkstoff in dem Solubilisator, gegebenenfalls unter Erwärmen, dispergiert wird und unter Rühren mit Wasser vermischt wird.

Gegenstand der Erfindung sind daher auch pharmazeutische Zubereitungen, die mindestens einen der erfindungsgemäßen Copolymere als Solubilisator enthalten. Bevorzugt sind solche Zubereitungen, die neben dem Solubilisator einen in Wasser schwerlöslichen oder wasserunlöslichen pharmazeutischen Wirkstoff, beispielsweise aus den oben genannten Indikationsgebieten enthalten.

Besonders bevorzugt sind von den oben genannten pharmazeutischen Zubereitungen solche, bei denen es sich um parenteral applizierbare Formulierungen handelt.

Der Gehalt an erfindungsgemäßem Solubilisator in der pharmazeutischen Zubereitung liegt, abhängig vom Wirkstoff, im Bereich von 1 bis 50 Gew.-%, bevorzugt 3 bis 40 Gew.-%, besonders bevorzugt 5 bis 30 Gew.-%.

### Solubilisatoren für Lebensmittelzubereitungen:

Neben der Anwendung in der Kosmetik und Pharmazie eignen sich die erfindungsgemäßen Copolymeren auch als Solubilisatoren im Lebensmittelbereich für schwer wasserlösliche oder wasserunlösliche Nähr-, Hilfs- oder Zusatzstoffe, wie z.B. fettlösliche Vitamine oder Carotinoide. Als Beispiele seien klare, mit Carotinoiden gefärbte Getränke genannt.

### Solubilisatoren für Pflanzenschutzzubereitungen:

Die Anwendung der erfindungsgemäßen Copolymere als Solubilisatoren in der Agrochemie kann u.a. Formulierungen umfassen, die Pestizide, Herbizide, Fungizide oder Insectizide enthalten, vor allem auch solche Zubereitungen von Pflanzenschutzmitteln, die als Spritz- oder Gießbrühen zum Einsatz kommen.

Gegenstand der Erfindung sind außerdem Copolymere, enthaltend
a) 85 bis 95 Mol-% mindestens einer monoethylenisch ungesättigten C₃-C₈-Carbonsäure,
b) 0,1 bis 15 Mol-% mindestens eines Monomeren ausgewählt aus der Gruppe
   b₁) N-C₁₂-C₂₂-Alkyl-substituierter Amide von monoethylenisch ungesättigten C₃-C₈-Carbonsäuren,
   b₂) N,N-C₁₂-C₂₂-Dialkyl-substituierter Amide von monoethylenisch ungesättigten C₃-C₈-Carbonsäuren,
c) 0 bis 14,9 Mol-% mindestens eines Monomeren ausgewählt aus der Gruppe der
   c₁) Vinylester von aliphatischen C₈-C₁₈-Carbonsäuren,
   c₂) C₈-C₁₈-Alkyl-Vinylether.

Für die allgemeine und bevorzugte Definition der einzelnen Monomerbausteine a) bis c) sowie für die Zusammensetzung und das Molekulargewicht der erfindungsgemäßen Copolymere ist die bereits eingangs erfolgte Beschreibung heranzuziehen.

Die Copolymeren besitzen K-Werte von mindestens 7, vorzugsweise 10 bis 30, besonders bevorzugt 10 - 25. Die K-Werte werden bestimmt nach H. Fikentscher, Cellulose-Chemie, Band 13, 58 bis 64 und 71 bis 74 (1932) in wäßriger Lösung bei 25°C, bei Konzentrationen, die je nach K-Wert-Bereich zwischen 0,1% und 5% liegen.

Die Copolymerisate werden dadurch hergestellt, daß man die entsprechenden Monomeren radikalisch polymerisiert.

Die Herstellung erfolgt nach bekannten Verfahren, z.B. der Lösungs-, Fällungs-, oder umgekehrte Suspensionspolymerisation unter Verwendung von Verbindungen, die unter den Polymerisationsbedingungen Radikale bilden.

Die Polymerisationstemperaturen liegen üblicherweise in dem Bereich von 30 bis 200, vorzugsweise 40 bis 110°C. Geeignete Initiatoren sind beispielsweise Azo- und Peroxyverbindungen sowie die üblichen Redoxinitiatorsysteme, wie Kombinationen aus wasserstoffperoxid und reduzierend wirkenden Verbindungen, z.B. Natriumsulfit, Natriumbisulfit, Natriumformaldehydsulfoxilat und Hydrazin.

Als Reaktionsmedium finden alle üblichen Lösungsmittel Verwendung, in denen die Monomere löslich sind. Vorzugsweise werden alkoholische Lösungsmittel wie z.B. Methanol, Ethanol, n-Propanol oder Isopropanol eingesetzt.

Um zu gewährleisten, daß die Reaktionen zu homogenen Produkten führen, ist es vorteilhaft, die Monomere und den Starter separat der Reaktionslösung zuzuführen. Dies kann beispielsweise in Form von getrennten Zuläufen für die einzelnen Reaktionspartner erfolgen.

Der Feststoffgehalt der erhaltenen organischen Lösung beträgt üblicherweise 20 bis 60 Gew.-%, insbesondere 25 bis 40 Gew.-%.

Das für die Polymerisation verwendete Lösungsmittel kann anschließend mittels Wasserdampfdestillation entfernt und gegen Wasser ausgetauscht werden.

Die wäßrigen Lösungen der Copolymere können durch verschiedene Trocknungsverfahren wie z.B. Sprühtrocknung, Fluidized Spray Drying, Walzentrocknung oder Gefriertrocknung in Pulverform überführt werden, aus der sich durch Redispergieren in Wasser erneut eine wäßrige Dispersion bzw. Lösung herstellen läßt.

In den folgenden Beispielen wird die Herstellung und Verwendung der erfindungsgemäßen Copolymere näher erläutert.

### Beispiel 1

Herstellung eines Acrylsäure/N-Octadecylacrylamid Copolymers (92/8 Mol-%)

In einem mit Stickstoff gespülten 2 Liter-Glaskolben mit vier getrennten Zulaufvorrichtungen wurden 10 g Acrylsäure in 250 g Isopropanol vorgelegt. Zulauf 1 enthielt 55 g N-Octadecylacrylamid in 300 g Isopropanol, Zulauf 2 enthielt 131 g Acrylsäure in 100 g Isopropanol, Zulauf 3 enthielt 9 g tert. Butylperpivalat (75%ige Lösung) in 50 g Isopropanol und Zulauf 4 enthielt 2,5 g tert. Butylperpivalat (75%ig) in Isopropanol. Die Vorlage wurde weiter mit Stickstoff gespült und unter Rühren auf 80°C erwärmt. Zulauf 1 wurde in 5,5 Stunden zugegeben, Zulauf 2 in 6 Stunden und Zulauf 3 in 6,5 Stunden. Nach einer weiteren Stunde wurde Zulauf 4 in einer Stunde zugegeben. Man ließ eine Stunde lang nachpolymerisieren. Anschließend wurde das Isopropanol durch Wasserdampfdestillation gegen Wasser ausgetauscht. Die wäßrige Suspension wurde mit Natronlauge auf pH 7 eingestellt. Man erhielt eine klare Lösung des Polymers mit einem Feststoffgehalt von 16,9%.

### Kosmetische Formulierungen

### Beispiel 2

6 g Solubilisator, hergestellt nach den Beispielen 1 und 2 wurden mit jeweils 1 g der in Tabelle 1 aufgeführten etherischen Öle bzw. Parfümöle mittels Magnetrührer innig gemischt. Unter ständigem Rühren wurde mit einer Bürette langsam demineralisiertes Wasser ad 100 g hinzugefügt. Die erhaltenen Formulierungen besaßen folgende Zusammensetzung:
1 Gew.-% etherisches oder kosmetisches Öl,
6 Gew.-% Solubilisator,
93 Gew.-% Wasser

**Tabelle 1**

| Solubilisator | etherisches Öl | Aussehen der Formulierung |
|---|---|---|
| | | |
| Beispiel 1 | Latschenkieferöl | klares Solubilisat |
| Beispiel 1 | Lavendelöl | opales Solubilisat |

### Beispiel 3

### Sonnenschutzmittel

25 g Copolymer, hergestellt gemäß Beispiel 1 wurden bei ca. 60°C in einer Mischung von 62,5 g Aqua bidest und 10 g Glycerol gelöst und die Lösung mit 2,5 g des Sonnenschutzmittels Uvinul® T 150 (Fa. BASF) bei 60°C unter schwachem Rühren versetzt. Es bildete sich eine klare Lösung, die nach Erkalten auf Raumtemperatur abgefüllt wurde.

### Beispiel 4

### Solubilisierende Wirkung am Beispiel Diazepam

Es wurden jeweils 1 Gew.-%ige Polymerlösungen der in Tabelle 2 genannten Copolymere (in Phosphatpuffer pH 7) mit überschüssigem Diazepam versetzt. Unter ständigem Schütteln stellte sich ein Gleichgewicht zwischen Polymer, Wirkstoff und Wasser ein. Der überschüssige Feststoff wurde abfiltriert. Im Filtrat wurde der Gehalt an Wirkstoff bestimmt.

**Tabelle 2**

| Copolymer | Solubilisierung |
|---|---|
| Acrylsäure/N-Stearylacrylamid (90 : 10 Gew.-%), Na-Salz | 160 µg/ml Diazepam 160 µg/ml Diazepam |
| Acrylsäure/N-Stearylacrylamid (90 : 10 Gew.-%), K-Salz | 269 µg/ml Diazepam 269 µg/ml Diazepam |
| Acrylsäure/N-Stearylacrylamid (80,9 : 19,1 Gew.-%), Na-Salz | 317 µg/ml Diazepam 317 µg/ml Diazepam |
| Acrylsäure/N-Stearylacrylamid (80,9 : 19,1 Gew.-%), K-Salz | 222 µg/ml Diazepam 222 µg/ml Diazepam |
| Acrylsäure/N-Stearylacrylamid (62,5 : 37,5 Gew.-%), Na-Salz | 433 µg/ml Diazepam 433 µg/ml Diazepam |
| Acrylsäure/N-Stearylacrylamid (66,8 : 33,2 Gew.-%), Na-Salz | 436 µg/ml Diazepam 436 µg/ml Diazepam |
| Acrylsäure/N-Stearylacrylamid (73,5 : 26,5 Gew.-%), Na-Salz | 375 µg/ml Diazepam 375 µg/ml Diazepam |
| Acrylsäure/N-Stearylacrylamid (66,7 : 33,3 Gew.-%), Na-Salz | 361 µg/ml Diazepam |
| | |
| Vergleich | |
| | |
| Phosphatpuffer, pH 7,0 | 50 µg/ml Diazepam |

### Beispiel 5

### Solubilisierende Wirkung am Beispiel 17-β-Estradiol und Clotrimazol

17-β-Estradiol und Clotrimazol wurden jeweils im Überschuß zu einer, auf 65°C erwärmten, 10 Gew.-%igen Polymerlösung [Acrylsäure/N-Stearylacrylamid Copolymer, (90:10 Gew.-%) in Phosphatpuffer pH 7,0] gegeben und bis zur Einstellung des Gleichgewichts langsam gerührt. Der überschüssige Feststoff wurde abfiltriert und der Gehalt an gelöstem Wirkstoff im Filtrat bestimmt (siehe Tabelle 3).

**Tabelle 3**

| Copolymer | Solubilisierung [Gew.-%] | |
|---|---|---|
| | | |

| | 17-β-Estradiol | Clotrimazol |
|---|---|---|
| Acrylsäure/N-Stearylacrylamid (90:10 Gew.-%), Na-Salz | 0,14 0,14 | 0,24 0,24 |
| | | |

| Vergleich: | | |
|---|---|---|
| | | |
| Phosphatpuffer, pH 7,0 | 0,0 | 0,0 |
| Tween® 80 | 0,045 | 0,015 |
| Cremophor® EL | 0,03 | 0,05 |

### Beispiel 6

### Diazepam Injektionslösung

250 mg Acrylsäure/N-Stearylacrylamid Copolymer (66,8 : 33,2 Gew.-%), Na-Salz wurden in 1728 mg Aqua bidest gelöst. Anschließend wurden 10 mg Diazepam zu der Solubilisatorlösung gegeben und gerührt bis der Arzneistoff gelöst war. Die Lösung wurde mit 2 mg Natriumdisulfit und 10 mg Benzylalkohol konserviert und nach üblichen Methoden sterilfiltriert und in Injektionsfläschchen gefüllt.

### Beispiel 7

### 17-β-Estradiol Gelatine Kapseln

100 mg 17-β-Estradiol wurden mit 10 g Acrylsäure/N-Stearylacrylamid - Copolymer (90 : 10 Gew.-%), Na-Salz, 80 g geschmolzenem PEG 6000 und 10 g Ethanol gemischt und anschließend direkt in flüssiger Form in Kapseln gefüllt.

### Beispiel 8

### Diazepam Emulsion zur parenteralen Applikation

80 g Acrylsäure/N-Stearylacrylamid Copolymer (66,8 : 33,2 Gew.-%) wurden in 720 g Aqua bidest gelöst. 10 g Diazepam wurden in einer 1: 1 Mischung aus Sojaöl und Miglyol Öl (Ölphase betrug 200 g) dispergiert. Zusätzlich wurden 10 g Sojalecithin eingesetzt, das in der Ölphase gelöst wurde. Die beiden Phasen wurden vordispergiert und anschließend per Hochdruckhomogenisation emulgiert.

### Beispiel 9

### 17-β-Estradiol Tablette

10 g 17-β-Estradiol wurden mit 200 g Acrylsäure/N-Stearylacrylamid - Copolymer (90 : 10 Gew.-%) und 290 g Ludipress® (Fa. BASF) sowie 0,5 g Mg-stearat gemischt. Die Mischung wurde anschließend direkttablettiert.

## Patentansprüche

1. Copolymere nach Anspruch 1, enthaltend
a) 85 bis 95 Mol-% mindestens einer monoethylenisch ungesättigten C₃-C₈-Carbonsäure,
b) 0,1 bis 15 Mol-% mindestens eines Monomeren ausgewählt aus der Gruppe
b₁) N-C₁₂-C₂₂-Alkyl-substituierter Amide von monoethylenisch ungesättigten C₃-C₈-Carbonsäuren,
b₂) N,N-C₁₂-C₂₂-Dialkyl-substituierter Amide von monoethylenisch ungesättigten C₃-C₈-Carbonsäuren,
c) 0 bis 14,9 Mol-% mindestens eines Monomeren ausgewählt aus der Gruppe der
c₁) Vinylester von aliphatischen C₈-C₁₈-Carbonsäuren,
c₂) C₈-C₁₈-Alkyl-Vinylether.
wobei sich die Mol-% Angaben der Einzelkomponenten zu 100% addieren.

2. Copolymere nach den Anspruch 1 mit einem Molekulargewicht von 1000 bis 30000 g/mol.

3. Verwendung von Copolymeren nach den Ansprüchen 1 oder 2 als Solubilisatoren.

4. Verwendung von Copolymeren nach Anspruch 3 als Solubilisatoren in kosmetischen Zubereitungen.

5. Kosmetische Zubereitungen, enthaltend mindestens eines der Polymere gemäß den Ansprüchen 1 oder 3.

6. Zubereitungen nach Anspruch 5, enthaltend einen in Wasser schwerlöslichen oder wasserunlöslichen kosmetischen Wirkstoff.

7. Verwendung von Copolymeren nach Anspruch 3 als Solubilisatoren in pharmazeutischen Zubereitungen.

8. Verwendung von Copolymeren nach Anspruch 7 mit einem Molekulargewicht von 1000 bis 30000 g/mol.

9. Pharmazeutische Zubereitungen, enthaltend mindestens eines der Polymere, gemäß den Ansprüchen 1 oder 2.

10. Pharmazeutische Zubereitungen nach Anspruch 9, wobei die Copolymere ein Molekulargewicht von 1000 bis 30000 g/mol besitzen.

11. Zubereitungen nach einem der Ansprüche 9 oder 10, enthaltend einen in Wasser schwerlöslichen oder wasserunlöslichen pharmazeutischen Wirkstoff.

12. Zubereitungen nach einem der Ansprüche 9 bis 11, **dadurch gekennzeichnet, daß** es sich um parenteral applizierbare Darreichungsformen handelt.

13. Verfahren zur Herstellung von Copolymeren gemäß den Ansprüchen 1 und 2, **dadurch gekennzeichnet, daß** man die Komponenten a) bis c) radikalisch polymerisiert.

14. Verfahren nach Anspruch 13, **dadurch gekennzeichnet, daß** man die Komponenten a) bis c) getrennt der Polymerisationsreaktion zuführt.

## Claims

1. A copolymer according to claim 1, comprising
a) 85 to 95 mol% of at least one monoethylenically unsaturated C₃-C₈-carboxylic acid;
b) 0.1 to 15 mol% of at least one monomer selected from the group
b₁) N-C₁₂-C₂₂-alkyl-substituted amides of monoethylenically unsaturated C₃-C₈-carboxylic acids,
b₂) N,N-C₁₂-C₂₂-dialkyl-substituted amides of monoethylenically unsaturated C₃-C₈-carboxylic acids,
c) 0 to 14.9 mol% of at least one monomer selected from the group of
c₁) vinyl esters of aliphatic C₈-C₁₈-carboxylic acids,
c₂) C₈-C₁₈-alkylvinyl ethers,
the mol% data for the individual components adding up to 100%.

2. The copolymer according to claim 1 having a molecular weight of from 1000 to 30,000 g/mol.

3. The use of copolymers according to claim 1 or 2 as solubilizers.

4. The use of copolymers according to claim 3 as solubilizers in cosmetic preparations.

5. A cosmetic preparation comprising at least one of the polymers according to claim 1 or 3.

6. The preparation according to claim 5 comprising a cosmetic active ingredient which is insoluble or virtually insoluble in water.

7. The use of copolymers according to claim 3 as solubilizers in pharmaceutical preparations.

8. The use of copolymers according to claim 7 having a molecular weight of from 1000 to 30,000 g/mol.

9. A pharmaceutical preparation comprising at least one of the polymers according to claim 1 or 2.

10. A pharmaceutical preparation according to claim 9, wherein the copolymers have a molecular weight of from 1000 to 30,000 g/mol.

11. The preparation according to claim 9 or 10 comprising a pharmaceutical active ingredient which is insoluble or virtually insoluble in water.

12. The preparation according to any of claims 9 to 11 in a parenterally administrable presentation.

13. The process for the preparation of copolymers according to claims 1 and 2 which comprises radically polymerizing the components a) to c).

14. The process according to claim 13 wherein the components a) to c) are introduced separately to the polymerization reaction.

## Revendications

1. Copolymères selon la revendication 1, contenant :
a) 85 à 95% en mole d'au moins un acide carboxylique en C₃-C₈ monoéthyléniquement insaturé;
b) 0,1 à 15% en mole d'au moins un monomère sélectionné parmi le groupe :
b₁) des amides substitués par un N-alkyle en C₁₂-C₂₂ d'acides carboxyliques en C₃-C₈ monoéthyléniquement insaturés,
b₂) des amides substitués par un N,N-dialkyle en C₁₂-C₂₂ d'acides carboxyliques en C₃-C₈ monoéthyléniquement insaturés;
c) 0 à 14,9% en mole d'au moins un monomère sélectionné parmi le groupe :
c₁) des vinylesters d'acides carboxyliques en C₈-C₁₈ aliphatiques,
c₂) des vinyléthers d'alkyle en C₈-C₁₈,
les données en % en mole des différents composants faisant 100%.

2. Copolymères selon la revendication 1 présentant un poids moléculaire de 1000 à 30 000 g/mole.

3. Utilisation de copolymères selon les revendications 1 ou 2 comme agents de solubilisation.

4. Utilisation de copolymères selon la revendication 3 comme agents de solubilisation dans des préparations cosmétiques.

5. Préparations cosmétiques contenant au moins un des polymères selon les revendications 1 ou 3.

6. Préparations selon la revendication 5, contenant une substance active cosmétique difficilement soluble dans l'eau ou insoluble dans l'eau.

7. Utilisation de copolymères selon la revendication 3 comme agents de solubilisation dans des préparations pharmaceutiques.

8. Utilisation de copolymères selon la revendication 7, avec un poids moléculaire de 1000 à 30 000 g/mole.

9. Préparations pharmaceutiques, contenant au moins un des polymères, selon les revendications 1 ou 2.

10. Préparations pharmaceutiques selon la revendication 9, les copolymères présentant un poids moléculaire de 1000 à 30 000 g/mole.

11. Préparations selon l'une des revendications 9 ou 10, contenant une substance active difficilement soluble dans l'eau ou insoluble dans l'eau.

12. Préparations selon l'une des revendications 9 à 11, **caractérisées en ce qu'**il s'agit de formes d'administration applicables par voie parentérale.

13. Procédé de fabrication de copolymères selon les revendications 1 et 2, **caractérisé en ce qu'**on polymérise les composants a) à c) par voie radicalaire.

14. Procédé selon la revendication 13, **caractérisé en ce que** l'on conduit les composants a) à c) à la réaction de polymérisation de manière séparée.
